# EUROPÄISCHE PATENTSCHRIFT

(11) **EP 2 886 131 B1**
(45) Veröffentlichungstag und Bekanntmachung des Hinweises auf die Patenterteilung: **24.08.2016**
(21) Anmeldenummer: 14198148.0
(22) Anmeldetag: 16.12.2014
(51) Int. Cl.: A61K 9/00, A61K 47/02, A61K 9/06, A61K 47/36, A61K 31/00, A61K 31/155, A61K 31/16

(54) **Transparentes Gel**
Transparent gel
Gel transparent

(30) Priorität: 23.12.2013 DE 202013105935 U; 24.01.2014 DE 202014100305 U
(43) Veröffentlichungstag der Anmeldung: 24.06.2015
(73) Patentinhaber: Nawa-Heilmittel Gmbh, 90482 Nürnberg (DE)
(72) Erfinder: Riesinger, Thomas, 90482 Nürnberg (DE)
(74) Vertreter: Glück Kritzenberger Patentanwälte PartGmbB

(56) Entgegenhaltungen:
- WO-A1-2010/091661
- US-A- 5 079 010
- S J Shelke ET AL: "Topical Gel: A novel Approach for Development of Topical Drug Delivery System", , 19. November 2013 (2013-11-19), Seiten 2739-2763, XP55181236, Gefunden im Internet: URL:http://www.ijptonline.com/wp-content/u ploads/2013/12/2739-2763.pdf [gefunden am 2015-04-07]

## Beschreibung

### Technisches Gebiet

Die Erfindung bezieht sich auf ein transparentes Gel.

### Stand der Technik

Zinkoxid wird sowohl zur Prophylaxe als auch zur Behandlung von entzündlichen Veränderungen der Haut und des Unterhaut-Zellgewebes eingesetzt. Aus der GB 20 22 998 A ist beispielsweise eine pharmazeutische Zubereitung bekannt, die aus einer wässrigen Lösung von wenigstens einem metallischen Spurenelement oder einem Salz eines solchen Spurenelementes besteht, wobei der pH-Wert der Lösung auf einen Wert kleiner als 4 eingestellt ist. Die EP 363 696 A1 beschreibt eine pharmazeutische Zubereitung, die aus einer wässrigen Lösung von metallischen Spurenelementen in Form von Zink und Eisen sowie Schwefelsäure als physiologische Säure besteht. Die Lösung weist einen pH-Wert zwischen 3,0 und 2,0 auf und enthält Zink, Eisen und Schwefelsäure in bestimmten Anteilen.

WO 2010/091661 offenbart eine Behandlungslösung für Wunden beinhaltend Zink, Eisen, Polyhexanid und Salzsäure.

Der Begriff "*Herpes simplex*" bezeichnet eine durch Herpes-simplex-Viren hervorgerufene Virusinfektion. Umgangssprachlich wird für eine solche Infektion meist das Wort Herpes verwendet. Die Erreger von Herpes-simplex-Infektionen sind zwei verschiedene Virusspezies: das Herpes-simplex-Virus 1 (HSV-1) und das Herpes-simplex-Virus 2 (HSV-2). Sie zeigen hinsichtlich ihrer Krankheitsbilder und der Krankheitslokalisation geringfügige Abweichungen. Nach der Lokalisation der Krankheitssymptome werden klinisch verschiedene HSV-Infektionen bezeichnet, von denen der *Herpes simplex labialis* (Lippenherpes) und der *Herpes simplex genitalis* (Genitalherpes) häufig vorkommen.

Der Lippenherpes wird vorwiegend mit Aciclovir und Valaciclovir behandelt. Aciclovir wird neben der Tablettenform auch als Creme angeboten. Die bekannten pharmazeutischen Zubereitungen führen bei der Behandlung von Lippenherpes zwar häufig zu einem Stillstand der Entwicklung der Krankheitssymptome, können aber kein beschleunigtes Abheilen bewirken.

### Darstellung der Erfindung

Hier setzt die Erfindung an. Es soll eine Zusammensetzung aufgezeigt werden, durch die ein beschleunigtes Abheilen von Lippenherpes bewirkt wird. Diese Aufgabe wird erfindungsgemäß durch das transparente Gel gemäß Anspruch 1 gelöst. Weitere vorteilhafte Aspekte, Details und Ausgestaltungen der Erfindung ergeben sich aus den abhängigen Ansprüchen und der Beschreibung.

Ein Gel im Sinne der vorliegenden Erfindung ist ein feindisperses System aus einer festen und einer flüssigen Phase. Die feste Phase bildet dabei ein schwammartiges, dreidimensionales Netzwerk, dessen Poren durch eine Flüssigkeit gefüllt sind.

Die vorliegende Erfindung stellt ein transparentes Gel enthaltend zumindest einen Gelbildner und eine wässrige schwefelsaure Lösung enthaltend von 10 mg bis 50 mg Zink und 5 mg bis 35 mg Eisen, jeweils bezogen auf 1 kg Gel, zur Verfügung. Die wässrige Lösung weist einen pH-Wert zwischen 3,0 und 2,0 auf und enthält 100 mg bis 1000 mg Polyhexamethylenbiguanid (PHMB) bezogen auf 1 kg Gel.

Überraschenderweise hat sich gezeigt, dass das erfindungsgemäße Gel nicht nur einen Stillstand der Entwicklung der Krankheitssymptome, sondern ein beschleunigtes Abheilen der Krankheitssymptome von Lippenherpes bewirkt. Aufgrund seiner transparenten Eigenschaften ist das Gel auf den Herpesbläschen im Gegensatz zu Cremes nicht sichtbar und fällt nicht auf.

Das Antiseptikum Polyhexamethylenbiguanid (PHMB) wird auch als Polyhexanid oder Polyaminopropyl Biguanid bezeichnet und ist ein polymeres Biguanid mit antimikrobiellen Eigenschaften.

Bevorzugt enthält das Gel zusätzlich Dexpanthenol. Dexpanthenol besitzt juckreizlindernde, entzündungshemmende, wundheilungsfördernde und hautpflegende Eigenschaften und hat sich in Kombination mit dem erfindungsgemäßen Gel als besonders geeignet für die Beschleunigung des Abheilens der Krankheitssymptome von Lippenherpes gezeigt.

Gemäß einer weiteren bevorzugten Ausführungsform ist in dem Gel zusätzlich zumindest ein Konservierungsstoff enthalten, wobei als Konservierungsstoff bevorzugt ein oder mehrere Konservierungsstoffe ausgewählt aus der Gruppe bestehend aus Benzylalkohol, Methylchloroisothiazolinon und Methylisothiazolinon eingesetzt werden.

Bevorzugt enthält das Gel Zink in Form von Zinkchlorid und Eisen in Form von Eisenchlorid.

Besonders bevorzugt sind in dem Gel 150 mg bis 500 mg Polyhexamethylenbiguanid (PHMB) bezogen auf 1 kg Gel enthalten. Insbesondere bevorzugt sind in dem Gel 200 mg bis 300 mg Polyhexamethylenbiguanid (PHMB) bezogen auf 1 kg Gel enthalten.

Als Gelbildner wurden verschiedene Stoffe wie z.B. Hydroxypropyl Starch Phosphat oder Dehydroxanthan Gum untersucht. Experimentelle Tests haben gezeigt, dass Dehydroxanthan Gum als Gelbildner besonders gut geeignet ist. Es bildet ein formstabiles transparentes Gel, das ein angenehmes Gefühl auf der Haut bewirkt, ausreichend haftet und nach dem Trocknen keine Rückstände auf der Haut hinterlässt. Dehydroxanthan Gum ist hochviskos und thermostabil und besitzt den Vorteil, dass das Gel beim Auftragen stabil bleibt und nicht fließt.

Bevorzugt enthält das Gel 100 mg bis 160 mg Schwefelsäure bezogen auf 1 kg Gel. Diese Menge an Schwefelsäure ist zur Einstellung des gewünschten pH-Werts zwischen 3,0 und 2,0 geeignet.

Besonders bevorzugt enthält das Gel von 20 mg bis 40 mg Zink und 10 mg bis 30 mg Eisen, jeweils bezogen auf 1 kg Gel. Insbesondere bevorzugt enthält das Gel 30 mg Zink und 19 mg Eisen, jeweils bezogen 1 kg Gel. Die genannten bevorzugten Ausführungsformen bewirken ein weiter beschleunigtes Abheilen der Krankheitssymptome von Lippenherpes.

Das erfindungsgemäße Gel ist ausgezeichnet verträglich, zeichnet sich durch eine gute Schleimhautverträglichkeit aus und gewährleistet eine hohe Bioverfügbarkeit der metallischen Spurenelemente Zink und Eisen, so dass bereits bei geringen Dosierungen der angestrebte therapeutische Effekt erreicht wird.

Es hat sich gezeigt, dass die Kombination von metallischen Spurenelementen in Form von Zink und Eisen, sowie wässrige Schwefelsäure und ein Anteil an PHMB zu ganz überraschenden, unerwarteten Wirkungen führt, nämlich zu einem schnellen und vollständigen Abheilen der Krankheitssymptome von Lippenherpes.

Bei dem erfindungsgemäßen Gel kann Wasser in Form von destilliertem Wasser oder elektrolytisch demineralisierten Wasser verwendet werden.

Eine Ausführungsform der erfindungsgemäßen Zubereitung, mit der sehr gute Ergebnisse erzielt werden, weist Konzentrationen von 30 mg Zink in Form von Zinkchlorid und 19 mg Eisen in Form von Eisenchlorid je kg Gel auf.

Ein weiterer Faktor für die Wirksamkeit des erfindungsgemäßen Gels ist der pH-Wert. Für die angestrebte Wirkung liegt dieser im Bereich zwischen 3,0 und 2,0. Die Einstellung des pH-Wertes erfolgt durch Zugabe einer entsprechenden Menge an Schwefelsäure.

Das Gel gemäß der vorliegenden Erfindung kann neben den metallischen Spurenelementen Eisen und Zink auch nichtmetallische Spurenelemente beispielsweise der Gruppe Silizium, Jod und Fluor enthalten, die in der Zubereitung in einer physiologischen Menge oder einem Vielfachen hiervon enthalten sind.

Für die Herstellung der erfindungsgemäßen Zubereitung eignen sich grundsätzlich die unterschiedlichsten Verfahren. So kann beispielsweise die Herstellung in der Weise erfolgen, dass die metallischen Spurenelemente Eisen und Zink vorzugsweise in zerkleinerter Form oder in Pulverform mit Säure und Wasser in Lösung gebracht werden, wobei dann der pH-Wert der Lösung durch die zugegebene Menge an Wasser und durch die zugegebene Menge an Schwefelsäure eingestellt wird.

Weiterhin ist es auch möglich, die erfindungsgemäße Zubereitung dadurch herzustellen, dass wasserlösliche Metallverbindungen der metallischen Spurenelemente, nämlich Zinkchlorid und Eisenchlorid, in Wasser unter Zugabe von Schwefelsäure gelöst werden, wobei auch in diesem Fall der erforderliche pH-Wert durch die zugegebene Menge an Wasser und Schwefelsäure eingestellt wird.

Bevorzugt wird ein Gel gemäß der vorliegenden Erfindung durch ein Verfahren hergestellt umfassend die Schritte Bereitstellen einer wässrigen schwefelsauren Lösung enthaltend 10 mg bis 50 mg Zink, 5 mg bis 35 mg Eisen und 100 mg bis 1000 mg Polyhexamethylenbiguanid (PHMB), jeweils bezogen auf 1 kg Gel, wobei die wässrige Lösung einen pH-Wert zwischen 3,0 und 2,0 aufweist, und unter Rühren Zugabe eines Gelbildners zur der bereitgestellten wässrigen schwefelsauren Lösung. Besonders bevorzugt wird zusätzlich der Schritt Zugabe eines Konservierungsstoffes zu der erhaltenen Lösung durchgeführt.

### Wege zur Ausführung der Erfindung

Zur Herstellung eines Gels gemäß der vorliegenden Erfindung werden zunächst 30 mg Zink in Form von Zinkchlorid und 19 mg Eisen in Form von Eisenchlorid in demineralisiertem Wasser gelöst. Anschließend werden 150 mg Polyhexamethylenbiguanid (PHMB) und Dexpanthenol zugegeben. Nach jeder Zugabe wird die Lösung mit Hilfe von verdünnter Schwefelsäure auf den gewünschten pH-Wert von 2,5 eingestellt. Anschließend wird der Gelbildner Dehydroxanthan Gum unter langsamen Rühren in die wässrige Lösung eingebracht. Nach Ausbildung des Gels in der gewünschten Form wird Methylchloroisothiazolinon als Konservierungsstoff zur verbesserten Haltbarkeit des Gels zugegeben.

## Patentansprüche

1. Transparentes Gel enthaltend zumindest einen Gelbildner und eine wässrige schwefelsaure Lösung enthaltend 10 mg bis 50 mg Zink und 5 mg bis 35 mg Eisen, jeweils bezogen auf 1 kg Gel, wobei die wässrige Lösung einen pH-Wert zwischen 3,0 und 2,0 aufweist, **dadurch gekennzeichnet, dass** 100 mg bis 1000 mg Polyhexamethylenbiguanid (PHMB) bezogen auf 1 kg Gel enthalten sind.

2. Gel nach Anspruch 1, **dadurch gekennzeichnet, dass** zusätzlich Dexpanthenol enthalten ist.

3. Gel nach Anspruch 1 oder 2, **dadurch gekennzeichnet, dass** zusätzlich zumindest ein Konservierungsstoff enthalten ist, wobei als Konservierungsstoff bevorzugt ein oder mehrere Konservierungsstoffe ausgewählt aus der Gruppe bestehend aus Benzylalkohol, Methylchloroisothiazolinon und Methylisothiazolinon enthalten sind.

4. Gel nach einem der Ansprüche 1 bis 3, **dadurch gekennzeichnet, dass** Zink in Form von Zinkchlorid und Eisen in Form von Eisenchlorid enthalten sind.

5. Gel nach einem der Ansprüche 1 bis 4, **dadurch gekennzeichnet, dass** 150 mg bis 500 mg Polyhexamethylenbiguanid (PHMB) bezogen auf 1 kg Gel enthalten sind.

6. Gel nach einem der Ansprüche 1 bis 5, **dadurch gekennzeichnet, dass** 200 mg bis 300 mg Polyhexamethylenbiguanid (PHMB) bezogen auf 1 kg Gel enthalten sind.

7. Gel nach einem der Ansprüche 1 bis 6, **dadurch gekennzeichnet, dass** als Gelbildner Dehydroxanthan Gum enthalten ist.

8. Gel nach einem der Ansprüche 1 bis 7, **dadurch gekennzeichnet, dass** 100 mg bis 160 mg Schwefelsäure bezogen auf 1 kg Gel enthalten sind.

9. Gel nach einem der Ansprüche 1 bis 8, **dadurch gekennzeichnet, dass** die wässrige Lösung von 20 mg bis 40 mg Zink und 10 mg bis 30 mg Eisen, jeweils bezogen auf 1 kg Gel, enthält.

10. Gel nach Anspruch 9, **dadurch gekennzeichnet, dass** 30 mg Zink und 19 mg Eisen, jeweils bezogen auf 1 kg Gel, enthalten sind.

## Claims

1. A transparent gel containing at least a gelling agent and an aqueous sulphuric acid solution containing 10 mg to 50 mg of zinc and 5 mg to 35 mg of iron, respectively per 1 kg of gel, wherein the aqueous solution has a pH between 3.0 and 2.0, **characterized in that** 100 mg to 1000 mg of polyhexamethylene biguanide (PHMB) are contained per 1 kg of gel.

2. Gel according to claim 1, **characterized in that** additionally dexpanthenol is contained.

3. Gel according to claim 1 or claim 2, **characterized in that** additionally at least one preservative is contained, wherein as the preservative preferably one or more preservatives selected from the group consisting of benzyl alcohol, methylchloroisothiazolinone and methylisothiazolinone are contained.

4. Gel according to one of claims 1 to 3, **characterized in that** zinc is contained in the form of zinc chloride and iron is contained in the form of ferric chloride.

5. Gel according to one of claims 1 to 4, **characterized in that** 150 mg to 500 mg of polyhexamethylene biguanide (PHMB) are contained per 1 kg of gel.

6. Gel according to one of claims 1 to 5, **characterized in that** 200 mg to 300 mg of polyhexamethylene biguanide (PHMB) are contained per 1 kg of gel.

7. Gel according to one of claims 1 to 6, **characterized in that** dehydroxanthan gum is contained as the gelling agent.

8. Gel according to one of claims 1 to 7, **characterized in that** 100 mg to 160 mg of sulphuric acid are contained per 1 kg of gel.

9. Gel according to one of claims 1 to 8, **characterized in that** the aqueous solution contains 20 mg to 40 mg of zinc and 10 mg to 30 mg of iron, respectively per 1 kg of gel.

10. Gel according to claim 9, **characterized in that** it contains 30 mg of zinc and 19 mg of iron, respectively per 1 kg of gel.

## Revendications

1. Gel transparent contenant au moins un gélifiant et une solution aqueuse d'acide sulfurique contenant 10 mg à 50 mg de zinc et 5 mg à 35 mg de fer, respectivement pour 1 kg de gel, la solution aqueuse présentant une valeur de pH comprise entre 3,0 et 2,0, **caractérisé en ce que** 100 mg à 1000 mg de polyhexaméthylène biguanide (PHMB) pour 1 kg sont contenus.

2. Gel selon la revendication 1, **caractérisé en ce que** du dexpanthénol est contenu en outre.

3. Gel selon la revendication 1 ou 2, **caractérisé en ce qu'**au moins un agent conservateur est contenu en outre, un ou plusieurs agents conservateurs sélectionnés dans le groupe composé du benzyle alcool, du méthylchloroisothiazolinone et du méthylisothiazolinone sont contenus.

4. Gel selon une des revendications 1 à 3, **caractérisé en ce que** zinc est contenu sous forme de chlorure de zinc et fer est contenu sous forme de chlorure de fer.

5. Gel selon une des revendications 1 à 4, **caractérisé en ce que** 150 mg à 500 mg de polyhexamethylène biguanide (PHMB) sont contenus pour 1 kg de gel.

6. Gel selon une des revendications 1 à 5, **caractérisé en ce que** 200 mg à 300 mg de polyhexamethylène biguanide (PHMB) sont contenus pour 1 kg de gel.

7. Gel selon une des revendications 1 à 6, **caractérisé en ce qu'**est contenue comme gélifiant de la gomme de déhydroxanthane.

8. Gel selon une des revendications 1 à 7, **caractérisé en ce que** 100 mg à 160 mg d'acide sulfurique sont contenus pour 1 kg de gel.

9. Gel selon une des revendications 1 à 8, **caractérisé en ce que** la solution aqueuse contient 20 mg à 40 mg de zinc et 10 mg à 30 mg de fer respectivement pour 1 kg de gel.

10. Gel selon la revendication 9, **caractérisé en ce que** 30 mg de zinc 19 mg de fer sont contenus respectivement pour 1 kg de gel.
